# EUROPEAN PATENT SPECIFICATION

(11) **EP 2 361 313 B1**
(45) Date of publication and mention of the grant of the patent: **18.12.2013**
(21) Application number: 09741303.3
(22) Date of filing: 26.10.2009
(51) Int. Cl.: C12Q 1/68

(54) **METHOD FOR DISTINGUISHING BETWEEN THE SPECIES COFFEA ARABICA AND COFFEA CANEPHORA AND HYBRIDS THEREOF BASED ON THE CONCOMITANT ANALYSIS OF NUCLEAR AND CHLOROPLASTIC DNA POLYMORPHISMS.**
VERFAHREN ZUR UNTERSCHEIDUNG ZWISCHEN DEN SPEZIES COFFEA ARABICA UND COFFEA CANEPHORA UND HYBRIDEN DAVON AUF DER GRUNDLAGE DER GLEICHZEITIGEN ANALYSE VON POLYMORPHISMEN NUKLEÄRER UND CHLOROPLASTEN-DNA
PROCÉDÉ POUR DISTINGUER LES ESPÈCES COFFEA ARABICA ET COFFEA CANEPHORA ET DES HYBRIDES DE CELLES-CI SUR LA BASE DE L'ANALYSE CONCOMITANTE DE POLYMORPHISMES D'ADN NUCLÉAIRE ET CHLOROPLASTIQUE

(30) Priority: 27.10.2008 IT PD20080307
(43) Date of publication of application: 31.08.2011
(73) Proprietor: DNA Analytica S.R.L., 34127 Trieste (TS) (IT)
(72) Inventor: PALLAVICINI, Alberto, I-34100 Trieste (IT); GRAZIOSI, Giorgio, I-34100 Trieste (IT); TORNINCASA, Patrizia, I-33040 Campeglio di Faedis (IT)
(74) Representative: Gervasi, Gemma
(86) International application number: PCT/EP2009/064044
(87) International publication number: WO 2010/049373

(56) References cited:
- US-A1- 2007 204 366
- SPANIOLAS STELIOS ET AL: "Authentication of coffee by means of PCR-RFLP analysis and lab-on-a-chip capillary electrophoresis" JOURNAL OF AGRICULTURAL AND FOOD CHEMISTRY, vol. 54, no. 20, October 2006 (2006-10), pages 7466-7470, XP002538316 ISSN: 0021-8561
- CHENWEI LIN ET AL: "Coffee and tomato share common gene repertoires as revealed by deep sequencing of seed and cherry transcripts" THEORETICAL AND APPLIED GENETICS ; INTERNATIONAL JOURNAL OF PLANT BREEDING RESEARCH, SPRINGER, BERLIN, DE, vol. 112, no. 1, 1 December 2005 (2005-12-01), pages 114-130, XP019322122 ISSN: 1432-2242 -& DATABASE EMBL [Online] 17 November 2005 (2005-11-17), "CGN-49951 Seed of Late Development Stage Coffea canephora cDNA clone cccs46w25e1 5', mRNA sequence." XP002565564 retrieved from EBI accession no. EMBL:DV703395 Database accession no. DV703395
- LASHERMES P ET AL: "Use of random amplified DNA markers to analyse genetic variability and relationships of Coffea species" GENETIC RESOURCES AND CROP EVOLUTION, vol. 40, no. 2, 1993, pages 91-99, XP002538317 ISSN: 0925-9864
- HENDRE PRASAD SURESH ET AL: "Development of new genomic microsatellite markers from robusta coffee (Coffea canephora Pierre ex A. Froehner) showing broad cross-species transferability and utility in genetic studies" BMC PLANT BIOLOGY, vol. 8, April 2008 (2008-04), XP002538318 ISSN: 1471-2229

## Description

### Field of the invention

The present invention relates to a method for distinguishing between different coffee species, in particular the species *Coffea arabica* and *Coffea canephora* and hybrids thereof, based on the concomitant use of one nuclear and one plastid marker, the nuclear marker consisting of an expressed sequence of *locus* RM-00-018, either comprising or not comprising an insertion sequence, and the chloroplast marker consisting of a sequence of *locus* P-AC comprising a microsatellite. The invention also relates to isolated polynucleotides comprising said markers and the primers for their amplification.

### State of the art

Coffee is one of the most important products on the international market, on which the commercial trade of many countries is based. It is among the 5 most economically important agricultural products in the world and is an essential economic resource for some developing countries.

Although the *Coffea* genus includes over 100 species, only two are important from the commercial viewpoint, namely the self-fertile allotetraploid *Coffea arabica* (Arabica coffee) and the self-sterile diploid *Coffea canephora* (Robusta coffee). *Coffea arabica* gives a distinctly superior beverage and accounts for 70% of world production and Arabica coffees are actually sold at a price 2-3 times higher than Robusta coffees. There are therefore serious economic reasons for expecting coffee to have a guarantee of authenticity. Adulteration of Arabica coffee with Robusta coffee can be intentional or otherwise and undertaken at the different stages of the production chain, from the plantation (there are producers of one, the other or both species) to the beverage itself. Authentication of green coffee beans would be very useful to the coffee roasting companies while identification of the roasted whole or ground coffee beans would be of interest to sellers and to consumers.

In general, methods based on molecular techniques using genetic markers, such as microsatellites or VNTRs (variable number of tandem repeats) and ESTs (expressed sequence tag) are more effective and reliable than those based on phenotypical characteristics. Microsatellites are tandem repeats of 10-60 nucleotides (nt), extending for 1-20 Kb, scattered within the genome and are inherited in a Mendelian modality. The number of repeats can differ between individuals and such case is known as polymorphism. Microsatellites have already been used for the characterization of varieties and species of plants such as rice (Zhou Z et al., 1997 Theor Appl Gen 95:942-949), and Bermuda grass (Karaca M and Ince AG, 2008 Journal of Genetics 87(1):83-86).

ESTs are portions of transcribed and translated genes in so far as they are obtained by sequencing cDNA fragments derived from retrotranscription of mRNA containing the information for encoding a protein. Researching these particular sequences is therefore very interesting in that the polymorphisms within them directly account for the phenotypical differences between individuals. In particular, the polymorphisms within ESTs consist of SNPs (single nucleotide polymorphisms) which are single base mutations within the sequence (Soleimani VD et al., 2003 Plant Molecular Biology Reporter 21: 281-288) or microsatellites i.e. tandem repeats of 1-6 nt (Aggarwal R et al., 2006 Theor Appl Genet 18:359-372).

Modern technology has enabled the nuclear DNA content of different species of the *Coffea* genus to be estimated, mainly by flow cytometry. The amount of nuclear DNA present in diploid species fluctuates between 0.95 pg in *C. racemosa* and 1.78 pg in *C. humilis* (Cros J et al., 1995 Canadian Journal of Botany 73:14-20; Noirot M et al., 2003 Annals of Botany 92:709-714). Intermediate values are found in *C. liberica dewevrei* and in *C. pseudozanguebariae,* which possess respectively 1.42 and 1.13 pg of DNA (Barre P et al., 1996 Cytometry 24(1): 32-38). The nuclear genome of *C. canephora* is 1.54 pg, while that of *C. arabica,* the only tetraploid species, is 2.61 pg; this value does not correspond exactly to the sum of the two parental genomes. The average DNA content in diploid species is 0.75 pg per haploid genome which correspond to about 700 million base pairs.

In contrast, each chloroplast contains several circular DNA molecules of about 150 kDa. The chloroplast genome, exhibiting maternal inheritance, is also characterized by a high degree of conservation between not closely related species.

The chloroplast genome (cpDNA) of plants has been used in the molecular analysis of their evolution by virtue of several characteristics: 1) it is small in size and forms a plentiful constituent of cellular DNA; 2) it has been widely characterized at the molecular level; 3) the nucleotides forming its sequence have a relatively low mutation rate and so can be used in phylogenetic studies.

The chloroplast genome sequence of *Coffea arabica* is 155189 nt in length and includes a pair of inverted repeats of 25943 nt. Of the 130 genes present, 112 are single and 18 are duplicated in the inverted repeat. The coding region comprises 79 genes which code for proteins, 29 genes for transfer RNA, 4 genes for ribosomal RNA and 18 genes containing introns (three of which having three exons), as described by Samson N et al., 2007 Plant Biotechnology Journal 5(2): 339-353.

Adulteration of Arabica coffee with Robusta coffee can be intentional or otherwise and undertaken at the various stages of the production chain, from the plantation to the beverage itself. Other commonly used contaminants are cereals (grain, soya), chicory, date seeds, tamarind, coffee twigs, raw sugar, etc.

Authentication of coffee beans can be carried out on the green grains in order to protect importers and coffee roasting companies, and on the roasted whole or ground coffee beans to protect sellers and consumers.

The analytical methods for determining the authenticity of foods include techniques that utilize the chemical and physical properties of foods, such as: spectroscopy (UV, IR, visible, Raman), isotope analyses, chromatography, electronic nose, as well as methods that entail DNA and protein analysis such as PCR, ELISA (Enzyme-Linked Immunosorbent Assay), thermal analysis and chemometric evaluation (Reid LM et al., 2006 Trends in Food Science and Technology 17:344-353).

There are only a few studies conducted on coffee and the methods available for measuring adulteration are: digital imaging (Sano EE et al., 2003 Journal of Food Quality 26(2):123-134), thermal lens and pH (Fontes AS et al., 2006 Instr Sci And Technol 34:163-181), infrared spectroscopy and chemometrics (Briandet R et al., 1996 J Sci Food Agric 71(3):359-366), voltammogrametry/chemometrics (Schreyer SK e Mikkelsen SR, 2000 Sens Actuators B: Chemical: 71(1):147-153), microscopy (Smith SD, 2001 Soc Hist Med 14:171-197) and HPLC (High Performance Liquid Chromatography) (Prodoillet J et al., 1995 J AOAC Int, 78(3): 749-761). In the majority of cases these methods have not been applied to the analysis of commercial samples, neither are they applicable on a large scale at low cost.

Studies on the adulteration of coffee have been conducted mostly with the aim of distinguishing between *C. arabica* and *C. canephora,* using chemical parameters such as the content of various substances such as: diterpene alcohols (Frega N et al., 1995 Indust. Aliment. 34:705-708), sterols (Valdenebro MS et al., 1999 Analyst 124:999-1002), volatile components (Bicchi CP et al., 1997 J Agric Food Chem 45:4680-4686), metal content (Martin MJ et al., 1999 Food Chem 66:365-370), chlorogenic acid and caffeine (Martin MJ et al., 1998 Talanta 46:1259-1264), fatty acids (Alves RM et al., 2003 JAOCS 80:511-17), tocopherols and triglycerides (Gonzales AG et al., 2001 Food Chem 73:93-101) and amino acids (Casal S et al., 2001 J Agric Food Chem 51:6495-6501). All these methods are based on quantitative ratios since no compound has ever been found that is present in only one of the two species.

Authentication of foods by DNA-based techniques is still in the initial stages of development. A general method exists for distinguishing between fruiting plant species by the polymorphisms found in the chloroplast rbcL gene. This gene is highly conserved, even though it exhibits small variable regions that enable the amplification of short fragments containing point mutations (SNPs i.e. single nucleotide polymorphisms) useful for species identification (Clegg MT, 1993 PNAS 90:363-367; Woolfe M and Primrose S, 2004 Trends in Biotechnology 22(5): 222-226).

The only DNA-based method described in the literature for distinguishing between *C. arabica* and *C. canephora* is that of Spaniolas S et al., 2006 J Agric Food Chem 54(20):7466-7470 which uses the PCR-RFLP method. The method is based on a SNP within a 251 nt long portion of the chloroplast gene trnL-trnF containing a restriction site for the endonuclease *Psu*I*.* All Arabica coffees do not exhibit the restriction site and give rise to a single amplified product of 251 nt, whereas the Robusta samples do not exhibit the SNP and digestion with *Psu*I generates two fragments of 92 and 159 nt. However this method is relatively difficult as it requires not only a PCR amplification but also digestion with costly restriction enzymes.

### Summary

The present invention relates to a method for distinguishing between species of coffee, in particular *Coffea arabica* and *Coffea canephora* and hybrids thereof.

For this purpose suitable polynucleotide sequences were identified and isolated which, in the various coffee varieties (cultivars) or species, differ only by the number, comprised between 0 and n, where n is between 1 and 4, of repeat sequences, according to the present invention identified by SEQ ID NO 2 and SEQ ID NO 6, in nuclear DNA or chloroplast DNA respectively, at the two specific polymorphic *loci,* namely a nuclear locus known as RM-00-O18 (SEQ ID NO 1) and a chloroplast locus known as P-AC (SEQ ID NO 5).

A first aspect of the present invention therefore relates to a polymerase chain reaction (PCR)-based method consisting of identifying the two polymorphic *loci* RM-00-O18 of SEQ ID NO 1 and P-AC of SEQ ID NO 5, by utilizing the unique regions flanking the repeat sequences SEQ ID NO 2 and SEQ ID NO 6 comprised within said RM-00-O18 and P-AC regions, in order to distinguish between two *Coffea* species and hybrids thereof. Hence, the method for distinguishing between different species of coffee (*Coffea spp.)* and hybrids thereof object of the invention is a molecular method comprising the amplification of polymorphic sites defined by the repetition from 0 to n times, where n is between 1 and 4, of sequences SEQ I D NO 2 and SEQ ID NO 6.

A second aspect of the present invention relates additionally to isolated polynucleotides comprising a nuclear chromosome DNA sequence and isolated polynucleotides comprising a plastid chromosome DNA sequence in which said nuclear DNA sequence is an expressed sequence of *locus* RM-00-O18 (SEQ ID NO 1) comprising or not comprising the insertion sequence SEQ ID NO 2, and said plastid DNA sequence is a sequence of *locus* P-AC (SEQ ID NO 5) comprising a microsatellite, of which the sequence SEQ ID NO 6 is the repeat monomer. Furthermore, the invention extends to the sequences complementary to sequence SEQ ID NO 1 and to oligonucleotides of SEQ ID NO 2.

In accordance with a preferred embodiment, the present invention also relates to oligonucleotides derived from the nuclear sequence SEQ ID NO 1 and from the plastid sequence SEQ ID NO 5, or from their complementary sequences, having a length between 10 and 35 nucleotides (nt) to be used as primers of the polymerization chain reaction.

The polynucleotides derived from said two nuclear and plastid sequences are at least 36 nt in length, preferably between 40 and 50 nt, and are designed with the aim of being able to hybridize specifically with sequences SEQ ID NO 1 and SEQ I D NO 5 respectively or with their complementary sequences.

The oligonucleotides and polynucleotides of the invention exhibit some degree of polymorphism in the various *Coffea* species and can be used for distinguishing between the species *Coffea arabica* and *Coffea canephora* and plant materials of varying nature and unknown origin, by polymerase chain reaction (PCR). The PCR method enables the presence of the two species of *Coffea* to be quantified in a particular sample of plant material. In addition, the concomitant use of the nuclear DNA marker and the plastid DNA marker enables to distinguish and assess the presence of the hybrids of the two species of *Coffea arabica* and *Coffea canephora.*

A further aspect of the invention therefore relates to a method for distinguishing between the species *Coffea arabica* and *Coffea canephora* and hybrids thereof and plant materials of varying nature and unknown origin, by the use of PCR, characterized by employing a nuclear chromosome DNA sequence and a plastid chromosome DNA sequence in which the nuclear sequence is an expressed sequence in the *locus* RM-00-O18 and the plastid sequence is the sequence in the *locus* P-AC, preferably comprising a microsatellite.

In accordance with the aforementioned aspects, the invention also relates to the kits for amplifying nuclear chromosome DNA fragments comprising at least one pair of oligonucleotides, a *forward* oligo- and *reverse* oligo- primer for the PCR, derived from the RM-00-O18 sequence (SEQ ID NO 1) and at least one pair of oligonucleotides, a *forward* oligo- and *reverse* oligo- primer for the PCR, derived from the P-AC sequence (SEQ ID NO 5) and optionally comprises as control sequences (for amplification) the expressed nuclear sequence of *locus* RM-00-018 (SEQ ID NO 1 or fragments thereof) and the plastid sequence of *locus* P-AC (SEQ ID NO 5 or fragments thereof) which preferably comprises the microsatellite repeat sequence.

The kit and the method can be usefully employed for distinguishing between the species *Coffea arabica* and *Coffea canephora* and plant materials of various types by means of PCR, using the identified polynucleotides and relative amplification primers with several applications such as differentiating between various species, identification of hybrids, evaluation of sample mixing level, identification of adulteration, incorrect labelling and comparison between samples.

### Brief description of the figures

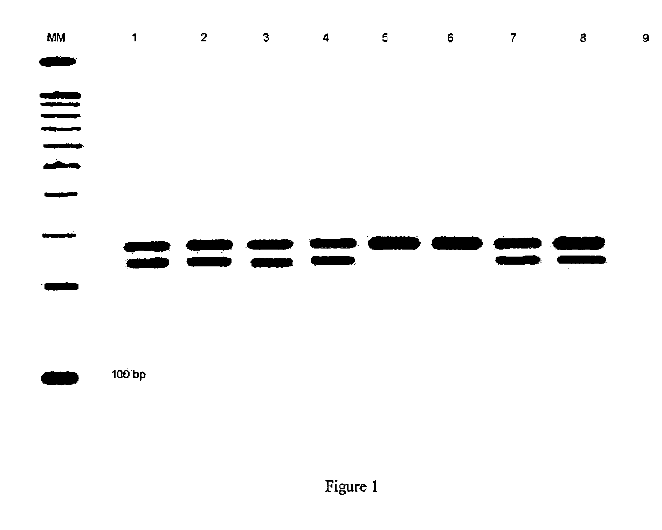

Figure 1 Analysis of *locus* RM-00-018. The bands represent the PCR amplification products of samples of *Coffea arabica* and *Coffea canephora* and mixtures of both species (Samples of different varieties of *Coffea arabica):* MM: Molecular weight marker of 100 nt; 1: *Coffea arabica* var. Aramosa; 2: *Coffea arabica* var. Caturra; *3: Coffea arabica* var. Laurina; 4: *Coffea arabica* var. Catuai; 5 and 6: Samples of *Coffea canephora* of different origin; 7: Amplification on a 50% *Coffea arabica* and 50% *Coffea canephora* blend; 8: Control of a 70% *Coffea arabica* and 30% *Coffea canephora* blend; 9: Negative control.

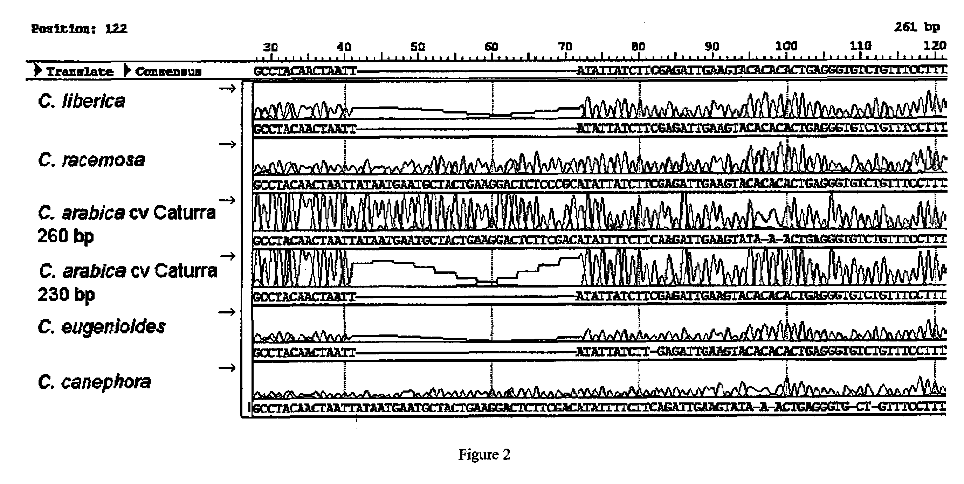

Figure 2 Sequencing of the upper band and lower band amplification product of *locus* RM-00-O18 of *C. arabica* and of the single bands present in *C. canephora* and in C. *eugenioides.* The upper band of Arabica has the same 30 nt insertion as the single band present in Robusta which is regarded as the male parent of Arabica. The lower band of Arabica has the same sequence as the corresponding band of C. *eugenioides* which is regarded as the female parent of C. arabica.

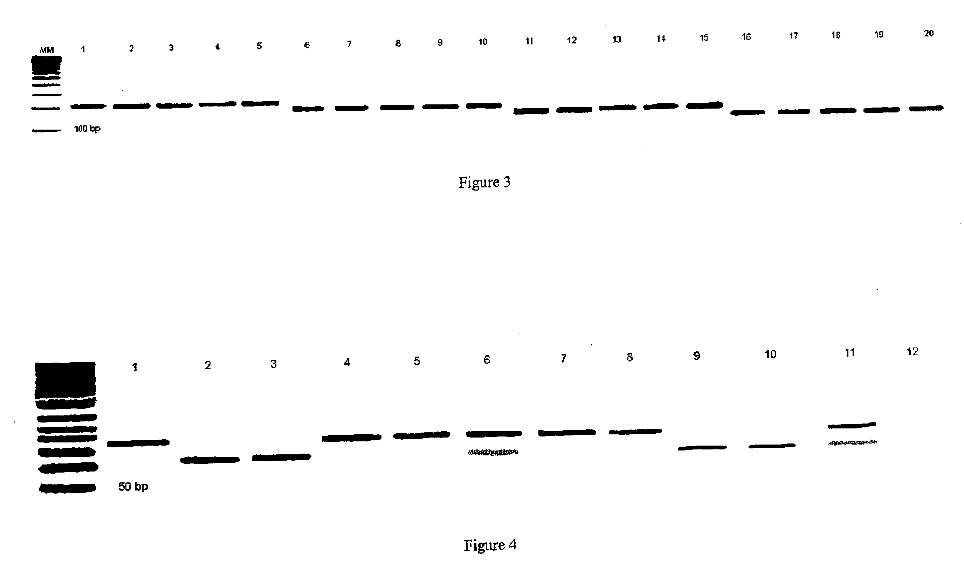

Figure 3 Comparison of the amplification products of plastid *locus* P-AC in Arabica coffees of different pedigrees and origins and in Robusta coffee. 1-5: Samples of *Coffea arabica:* Etypica, Tafarikela, Sln.5B, Yirga 4 and Yirga 8 with four monomer repeats in the amplification product of 201 nt; 6-10: Samples of *Coffea arabica:* Caturra, Bourbon VD, Catuai 144, Typica, HdT with three monomer repeats in the amplification product of 169 nt; 11-15: Samples of *Coffea arabica:* Agaro 1, Agaro 2 and Agaro 3 with two monomer repeats in the amplification product of 137 nt; 16-20: Different samples of *Coffea canephora* with a single monomer repeat in the amplification product of 105 nt; MM: Molecular weight marker of 100 nt.
Figure 4 Amplification products of the plastid *locus* in different roasted and ground commercial samples. MM: Molecular weight marker 50nt; 1: Control of *Coffea arabica;* 2 and 3: Control of *Coffea canephora;* 4-7: Commercial samples sold as *Coffea arabica;* 8-12: Commercial samples where the composition is not specified on the package.

### Detailed description of the invention

### Definitions

The standard IUPAC nomenclature was used to represent the nucleotides forming the sequences: K=G or T; Y=C or T; R=A or G; M=A or C; S=G or C; B=C, G or T; D=A, G or T; H=A, C or T and V=A, C or G. The abbreviation nt is herein used for "nucleotide/s".

The term "microsatellite" or "variable number of tandem repeats" (VNTR) is used herein in its commonly accepted significance to indicate sequences of 10-60 nucleotides repeated at least once in a certain DNA nucleotide sequence delimited by unique sequences. Said microsatellites can be represented by the general formula (NᵢNᵢ....Nᵢ)ₙ in which N represents the nucleotides A, T, C or G, i represents the number of repeated nucleotides and n is the number of times in which the VNTR motif is repeated (for example (G₂A₂C₃G₃)₅ represents a decanucleotide repeated 5 times in a given sequence). Different individuals can exhibit, at a specific *locus,* a different and unpredictable number of monomer repeats; the microsatellite in this case is defined as polymorphic. These polymorphisms are transmitted stably and can hence be used as molecular markers.

The term "EST" (Expressed Sequence tag) is used herein in its commonly accepted significance to indicate short DNA fragments obtained by retrotranscription of messenger RNAs, representing genes which are effectively transcribed and translated.

The term "polynucleotide" sequence means sequences greater than 35 nt in length; the definition also encompasses complementary sequences thereof.

The term "primers" or "triggers" or "oligonucleotides" is herein used in its current meaning in particular being a poly- or oligonucleotide sequence from the flanking regions or their complementary sequences, which identify unequivocally an identified *locus.* Primers are normally formed from at least 10 nucleotides (nt) and are preferably 18-35 nt in length. The nucleotide composition of the primers can vary and must be such as to satisfy the following characteristics: length between at least 10, 11, 12, 13, 14, 15, 16, 17, 18 nucleotides and 35 nucleotides; G+C content between 50 and 60%; melting temperature Tₘ between 54 and 60ºC; maximum Tₘ difference equal to 2ºC; non-formation of secondary structures, such as primer dimers; least possible complementarity at the 3' end. The amplification fragment length varies according to where it is most expedient to design the primers, always provided that the sequence of the latter is such as to be able to hybridize with the *locus* flanking sequences under stringent conditions.

The term *"locus"* means the location on a chromosome of a DNA sequence either repeated or not repeated that determines a polymorphism. In this sense the term *locus* is equivalent to a polymorphic site. A *locus* can be occupied by any of the alleles of a gene, an allele being all the alternative forms in which the same gene exists. Diploid or polyploid cells can be defined as homozygotes if they possess the same allele in the same *locus* in every chromosome, or heterozygotes if they possess different alleles in the corresponding *loci.*

The 5' and 3' flanking regions identify unequivocally the *locus* of an expressed sequence and a microsatellite within the genome. Different individuals can exhibit, at a specific microsatellite *locus* on a chromosome, a different number of monomer repeats (which in this case give rise to the possible alleles); the microsatellite in this case is defined as polymorphic. In the case of the expressed sequence the polymorphism is the same length as the amplified product, the length having been given by the presence or absence of a non-repeat insertion of nucleotides and in particular 30 nt as in the present invention. In particular the *locus* can be occupied by any allele of the sequence with or without the 30 nt insertion. These polymorphisms are stably transmitted and can hence be used as molecular markers. The term *locus* is herein used with reference to the location of the VNTR sequences and expressed sequences as well as the unique flanking regions from which the primers for amplification are derived.

The term genetic or molecular "markers" (nuclear or chloroplast) signifies those polynucleotides derived from nuclear chromosome DNA or plastid (chloroplast) chromosome DNA which comprise sequences able to give a certain degree of polymorphism. In particular, throughout the text the term "nuclear chromosome marker" means the sequence SEQ ID NO 1 of *locus* RM-00-O18, whose polymorphism is determined by the presence or absence of a 30 nucleotide insertion sequence SEQ ID NO 2, while the term "plastid chromosome marker" means the sequence SEQ ID NO 5 of *locus* P-AC comprising a microsatellite SEQ ID NO 6, whose polymorphism is determined by an unpredictable number of monomer repeats forming the microsatellite itself.

### Biological material: source and place of origin

The biological material used for identifying the nuclear and plastid chromosomal DNA markers of the invention have been isolated from the following varieties of *Coffea arabica,* interspecies hybrids thereof, *Coffea canephora* and *Coffea eugenioides* listed herein below.

| **Plant** | **Genealogy** | **Origin** |
|---|---|---|
| Typica | Variety of *C. arabica* | Costa Rica, CATIE |
| Bourbon VD | Mutant of Bourbon | Brazil, Patrocinio |
| Caturra | Mutant of Bourbon | Costa Rica, CATIE |
| Laurina | Mutant of Bourbon | Brazil, Patrocinio |
| Etypica | Ethiopian selection of C. *arabica* | Brazil, Patrocinio |
| Tafarikela Sln 4 | Wild selection of *C. arabica* | India, Karnataka Chikmagalur |
| Agaro-1 (1-24) | *C. arabica* from Ethiopia | Agaro, Ethiopia |
| Agaro-2 (1-24) | *C. arabica* from Ethiopia | Agaro, Ethiopia |
| Agaro-3 (1-24) | *C. arabica* from Ethiopia | Agaro, Ethiopia |
| Yirga (4, 8) | *C. arabica* from Ethiopia | Yirgacheffè, Ethiopia |
| Catuai 144 | Mundo Novo x Caturra | Brazil, Patrocinio |
| Catuai | Mundo Novo x Caturra | Brazil, Patrocinio |
| Aramosa | *C. arabica x C. racemosa* | Brazil, Patrocinio |
| Hibrido de Timor (HDT) | *C. arabica x C. canephora* | Costa Rica, CATIE |
| Sln.5B | S333 x Devamachy | India, Karnataka Chikmagalur |
| *C. canephora* T3481 t5 | Species | Costa Rica, CATIE |
| *C. canephora* T3483t1 | Species | Costa Rica, CATIE |
| *C. canephora* T3518t2 | Species | Costa Rica, CATIE |
| *C. canephora* T3563t2 | Species | Costa Rica, CATIE |
| *C. canephora* T3564t1 | Species | Costa Rica, CATIE |
| *C. eugenioides* | Species | Brazil, Campinas |

### Detailed description

The present invention relates to a molecular-type process useful for distinguishing between different coffee species (*Coffea* spp.) and in particular between *Coffea arabica* and *Coffea canephora* and hybrids thereof at each production stage, from the plant to the roasted coffee, it having multiple applications such as evaluating the sample mixing level or identifying adulteration, incorrect labeling and comparing samples.

With the aim of properly distinguishing between the *Coffea* species and any interspecies hybrids, it was necessary to identify robust, stable and easily detectable molecular markers.

Said markers were identified in polynucleotide sequences of chromosome DNA, one of which consisting of an expressed sequence of nuclear chromosome DNA comprising or not comprising a 30 nt insertion sequence, and the other consisting of a plastid chromosome DNA sequence comprising repeat sequences known as microsatellites. The presence or absence of the DNA insertion sequence in the identified nuclear *locus,* as well as the number of repetitions of the repeat DNA sequence VNTR of the plastid *locus* characterizes said polymorphic sites. For the purposes of the present invention polynucleotides comprising the identified markers have therefore been isolated and from these the specific poly- or oligonucleotide sequences were derived for use as primers for the molecular method of the invention and hence to amplify the genetic material of coffee of unknown taxonomic origin.

The first identified nuclear marker or polymorphic site is the nuclear chromosome *locus* RM-00-O18, whose polymorphism is based on the insertion or non insertion of a 30 base pair sequence of sequence 5'-ATAATGAATGCTACTGAAGGACTCTTCGAC-3' (SEQ ID NO 2). Said insertion sequence can be comprised within the polynucleotide of SEQ ID NO 1 from nucleotide 88 onwards of the sequence of the *locus* RM-00-O18 in question. In particular the nucleotide sequence of *locus* RM-00-O18 was isolated from cDNA libraries constructed from *Coffea arabica* leaf and root tissue.

Amplification of this *locus* enables Arabica and Robusta to be differentiated. In this respect amplification of the RM-00-O18 *locus* of *C. arabica* produces two bands of 230 nt and 260 nt, while *C. canephora* produces a single band of 260 nt.

On the polynucleotide sequence of the RM-00-O18 *locus,* with the exception of the insertion sequence, i.e. on the regions flanking the insertion sequence (SEQ ID NO 2) comprised within the polynucleotide of SEQ ID NO 1, there can be derived poly- and oligo-nucleotides of at least 10 nucleotides, preferably between 18 and 35 nt, for use as primers for amplifying DNA fragments of *locus* RM-00-O18 of said SEQ ID NO 1. In particular, primers for the amplification of DNA fragments comprising *locus* RM-00-O18, and possibly the insertion sequence ATAATGAATGCTACTGAAGGACTCTTCGAC (SEQ ID NO 2), are derived from any of the sequences comprised between the nucleotide in position 1 and the nucleotide in position 87, preferably between the nucleotide in position 27 and the nucleotide in position 47, for the *forward* primers 5'-3', whereas the *reverse* primers 5'-3' are derived from any of the sequences comprised between the nucleotide in position 88 and the nucleotide in position 261, preferably between the nucleotide in position 235 and the nucleotide in position 259 of SEQ ID NO 1. The primers are at least 10 nucleotides and preferably 18-35 nt in length. The nucleotide composition of the primers can vary although preferably satisfying the following characteristics: length comprised between at least 10, 11, 12, 13, 14, 15, 16, 17, 18 nucleotides and 35 nucleotides; G+C content between 50 and 60%; melting temperature Tₘ between 54 and 60ºC; maximum Tₘ difference equal to 2 ºC; non formation of secondary structures such as primer dimers; least possible complementarity at the 3' end.

The most preferred oligonucleotide primer sequences for amplification of the DNA fragments of *locus* RM-00-O18 of SEQ ID NO 1, either comprising or not comprising the insertion sequence ID NO 2 (SEQ ID NO 2), are identified hereinafter as SEQ ID NO 3 and SEQ. ID NO 4.

The second identified gene marker is in the plastid chromosome P-AC *locus,* whose polymorphism is based on the number of microsatellite repeats having a repeat sequence of the type TTTTCGTTTTCATATATTGAAAAAGTAATAAG (SEQ ID NO 6).

For the purposes of the present invention the polynucleotide comprising the microsatellite of *locus* P-AC was isolated from the plastid DNA of *C. arabica,* being preferably the polynucleotide identified as SEQ ID NO 5, in which the microsatellite is inserted after the nucleotide at position 392 and is repeated three times. The microsatellite is formed of a 32 nt monomer of SEQ ID NO 6 and the polymorphism consists of the different number of times in which said monomer is repeated. In particular, *C. arabica* can exhibit 2, 3 or 4 repeat monomers, while C. *canephora* always exhibits only one. For the purposes of the present invention therefore the number of microsatellite repeats of SEQ ID NO 6 is from at least one to at least 4.

Poly- and oligo-nucleotides of at least 10 nucleotides, preferably between 18 and 35 nt, can be designed on the polynucleotide sequence of *locus* P-AC with the exception of the repeat sequence forming the microsatellite, i.e. on the regions flanking the microsatellite of SEQ ID NO 6, for use as primers in the amplification of DNA fragments of the *locus* P-AC comprising the sequence which characterises the microsatellite (TTTTCGTTTTCATATATTGAAAAAGTAATAAG)*ₙ*, in which n is at least equal to 1. In particular, the primers for amplifying said DNA fragments are derived from any of the sequences comprised between the nucleotide at position 1 and the nucleotide at position 392 and preferably between the nucleotide at position 355 and the nucleotide at position 375 of SEQ ID NO 5 for the *forward* primers 5'-3-, whereas the *reverse* primers 5'-3' are derived from any of the sequences comprised between the nucleotide at position 393 and the nucleotide at position 864 and preferably between the nucleotide at position 408 and the nucleotide at position 427 of SEQ I D NO 5.

The primers in this case have the previously mentioned characteristics for primers suitable for amplifying nuclear chromosome DNA fragments of the polynucleotide of *locus* RM-00-O18 of SEQ ID NO 1.

The oligonucleotide sequences of the most preferred primers for amplifying DNA fragments of *locus* P-AC are identified in the following as SEQ ID NO 7 and SEQ ID NO 8.

The sequences of the isolated markers are given in table 1, while the sequences of the specific primers are given in table 2.

**Table 1: Gene markers**

| Marker | *Locus* | Sequence type / nucleotide position |
|---|---|---|
| | RM-00-O18 (SEQ ID NO 1) | Insertion / after nt 87 |
| nuclear | | (ATAATGAATGCTACTGAAGGACTCTTCGAC)*ₙ* (SEQ ID NO 2) |
| | | *n* = 0 or 1 |
| | P-AC (SEQ ID NO 5) | Microsatellite/ after nt 392 |
| chloroplast (cytoplasm) | | (TTTTCGTTTTCATATATTGAAAAAGTAATAAG)ₙ (SEQ ID NO 6) |
| | | *n* = from 1 to at least 4 |

**Table 2: Primers**

| *Locus* | Forward primers (5'-3') | Reverse primers (5'-3') |
|---|---|---|
| RM-00-O18 | TGGAAATGGTTAAGGCTGTT G | AGCTCCAAACAAGATTTTATTCCTT |
| | | (SEQ ID NO 4) / nt 235-259 |
| | (SEQ ID NO 3) / nt 27-47 | |
| P-AC | TTGGGGTTTTGAATAAGACGA | ACGCCAGGATGATAAAAAGC |
| | (SEQ ID NO 7) / nt 355-375 | (SEQ ID NO 8) / nt 408-427 |

The combined use of the two polymorphic *loci* enables species as well as hybrids thereof to be distinguished, in that whereas the nuclear DNA of a hybrid is always mixed between the two species, chloroplast DNA is only ever inherited from the maternal line. Frequently commercial coffees result from selection of the progenies of Arabica/Robusta hybrids and the combined use of the two polymorphic *loci* allows the consistency between nuclear DNA and plastid DNA to be evaluated. A case could arise where the polymorphic nuclear *locus* corresponds to Arabica and the polymorphic plastid *locus* corresponds to Robusta, if the hybrid has been crossed with Robusta as the female parent.

Said sequences have proved to be useful for developing a method for the genetic identification of the two most economically important *Coffea* species, namely *Coffea arabica* and *Coffea canephora* and hybrids thereof.

The developed method uses the polymerase chain reaction technique (PCR) which allows the multiplication (amplification) of nucleic acid fragments of known nucleotide sequences. PCR amplification enables the amount of genetic material required for subsequent applications to be very rapidly obtained *in vitro.* This process is well known and it is carried out by enzymes called DNA-polymerases able to progressively synthesize a new DNA strand under the following conditions, by putting into solution: a) an even minimal amount of the DNA segment required to be reproduced; b) a suitable amount of free nucleotides to form the new strands; c) suitable triggers, known as primers, consisting of short DNA sequences (oligonucleotides) complementary to the 5' and 3' ends of the segment to be reproduced; d) other support elements (e.g. magnesium ions) needed to form a suitable reaction environment); e) a DNA polymerase.

Briefly as well known to initiate the polymerase reaction, provision must firstly be made to separate the DNA strands (denaturation step), then to bind the primers to the complementary regions of the denatured DNA strands (annealing step) to end with elongation of the strand starting from the 5' primer (extension step). This enables several PCR cycles to be undertaken sequentially, within each of which the DNA synthesized in the preceding steps is also duplicated to hence achieve a chain reaction that allows an extremely rapid multiplication of the genetic material of interest.

As is known, PCR enables the reconstitution (synthesis) of a complete DNA segment (double helix) starting from a single helical strand. The missing strand is reconstructed from a series of nucleotides (the fundamental components constituting nucleic acids) which are positioned in the correct sequence, and are complementary to the DNA of interest.

This process is achieved in nature by DNA polymerase enzymes which are able to progressively synthesize a new DNA strand under certain conditions: polymerization nucleotides in the form of deoxyribonucleotide triphosphate (dNTP) must be available; the DNA must be denatured, or the two helices that comprise the strands must already be separated; the segment to be reconstructed can only be elongated, that is to say a new strand cannot be synthesized from zero; suitable conditions of temperature, pH etc. must be also respected.

This process is undertaken by polymerases belonging to thermophilic organisms which are not inactivated by high temperatures, for example Taq polymerase deriving from the thermophilic bacterium *Thermus aquaticus.* This enables several PCR cycles to be undertaken sequentially, within each of which the DNA synthesized in the preceding steps is also duplicated to hence produce a chain reaction that allows an extremely rapid multiplication of the genetic material of interest.

Therefore, according to the invention a method for distinguishing between different species of coffee (*Coffea spp.)* and hybrids thereof comprises at least the following steps:
a) amplification by means of a polymerase chain reaction of a DNA fragment from a coffee DNA sample by means of oligonucleotide primers comprised between nucleotides 1 and 261 of the sequence SEQ ID NO 1 (and by means of oligonucleotide primers comprised between nucleotides 1 and 864 of the sequence SEQ ID NO 5;
b) separation of the amplified fragment according to the amplification product molecular weight;
c) comparison of the previously identified molecular weight with that of the fragments generated from the same amplification type in the reference species/cultivar.

The primers for the amplification step are preferably the oligonucleotides of sequence SEQ I D NO 3 and SEQ ID NO 4 for *locus* RM-00-O18 and of sequence SEQ ID NO 7 and SEQ ID NO 8 for *locus* P-AC.

The species and cultivars of coffee, and in particular *Coffea arabica* and *Coffea canephora,* are differentiated by identifying, within the amplified product obtained by PCR using the appropriate primers for amplification of DNA fragments of the *locus* RM-00-O18 and P-AC, the presence or absence of at least one fragment characteristic of a determined species or cultivar of said *locus* RM-00-O18 and/or a difference in size of the same amplified product of said *locus* the P-AC, while the consistency between nuclear DNA and plastid DNA allows discrimination between possible hybrids. The method of the invention is applicable to different samples taken from coffee plants and in particular is applicable to parts of coffee plants chosen from leaves, flowers, fruit, stem, roots, whole green coffee seeds or single embryos, roasted coffee whole beans or ground beans and mixtures thereof.

### Experimental part

### Extraction of DNA from C. arabica and C. canephora

Genomic DNA was extracted from fresh or lyophilized young leaves, fresh or roasted beans using the technique based on CTAB (cetyltrimethylammonium bromide), modifying the protocol devised by Murray MG and Thompson WF, 1980 Nucl Acids Res 8: 4321-4325, and consists of the following operations:
- grind about 2.5 g of fresh tissue and place in a 50 ml test tube;
- homogenize under cold conditions in 25 ml of extraction buffer (Tris-HCl 50 mM pH 8.0, EDTA 5 mM, sorbitol 0.35 M, BSA 0.1%, PEG 6000 10%, PVP 40 1%) to which 25 µl of fresh β-mercaptoethanol have been added;

- filter the homogenate through several layers of gauze;
- centrifuge at 6000 x g for 15 minutes at 4°C;
- remove the supernatant and resuspend the pellet in 1.25 ml of washing buffer (Tris-HCl 50 mM pH 8.0, EDTA 5 mM, sorbitol 0.35 M) and 1.25 µl of β-mercaptoethanol;
- add 250 µl of 5% Sarcosile and hold for 15 minutes at room temperature;
- add 200 µl of 5 M NaCl and 112.5 µl of 10% CTAB;
- incubate at 37ºC overnight;
- extract with 1.850 ml of CHL:IAA (24:1 chloroform:isoamyl alcohol)
- transfer the upper aqueous phase into a 15 ml test tube;
- repeat the extraction;
- add 1.5 ml of isopropanol to the aqueous phase, carefully invert 2-3 times to precipitate the DNA;
- when visible, remove the precipitated DNA with a sterile rod or centrifuge at low speed;
- discard the supernatant and add 500 µl of 75% ethanol to the DNA;
- allow to desalinate for 20 minute, then remove the supernatant by completely evaporating the residual ethanol;
- add 100 µl of water, redissolve for at least 30 minutes at room temperature and transfer into a 1.5 ml Eppendorf tube.

DNA was also extracted from seeds using the method described by Chang S et al., 1993 Plant Molecular Biology Reporter 11: 113-116. This latter protocol, devised for the extraction of RNA, was used to extract DNA by substituting the LiCl with isopropanol for the precipitation.

In some cases the DNA was isolated from single embryos taken from the coffee bean in the following manner:
- place the seeds in water overnight to rehydrate them;
- extract the embryo with a blade to separate it from the endosperm;
- place the embryo at the base of a 1.5 ml tube;
- add 100 µl of CxG Bufffer and 5 µl CxG Enzyme mix (Promega) to each sample;
- incubate at 50ºC while continuously mixing;
- allow the digestion to proceed for 2-3 hours;
- add 200 µl of Red Blood Lysis Solution (Talent) and incubate for 30 minutes at 68ºC;
- if necessary extract with one volume of a 24:1 chloroform:isoamyl alcohol solution;
- purify the DNA using a Cleanmix kit (Talent) according to the manufacturer's instructions. This method is based on the selective binding of DNA to a high capacity matrix in the presence of a chaotropic agent (guanidine thiocyanate);
- quantify the extracted DNA using a fluorimeter.

### PCR amplification of locus RM-00-O18

In the specific case of *locus* RM-00-O18, the PCR reaction was prepared using the following reagents:

| *Reagent* | *Final concentration* |
|---|---|
| DNA | 20 ng |
| *Forward* primer (SEQ ID NO 3) | 200 nM |
| *Reverse* primer (SEQ ID NO 4) | 200 nM |
| MgCl₂ | 2 mM |
| dNTPs | 200 mM |
| PCR buffer (10 mM Tris-HCl pH 9.0, 50 mM KCI, 0.1% Triton X-100) | 1x |
| Taq DNA polymerase | 1 unit |
| H₂O | qb to 25 µl |

The amplification conditions comprise: initial denaturation at 94ºC (3 min), followed by 35 cycles of: denaturation at 94ºC (25 sec), annealing at 58ºC (30 sec) and extension at 72ºC (30 sec), ending with a final extension at 72ºC (10 min).

The amplification products were separated by electrophoresis on 2% agarose gel. The obtained results clearly show that *locus* RM-00-O18 enables the amplification of two bands in *Coffea arabica* (230 and 260 nt), a single band in *Coffea canephora* (260 nt) and a single band in *Coffea eugenioides* (230 nt), as shown in figure 1.

Figure 2 instead shows that this system is useful in identifying mixtures of Arabica coffee and Robusta coffee. The difference in length of the two bands is due to a 30 nt insertion (5'-ATAATGAATGCTACTGAAGGACTCTTCGAC-3') (SEQ ID NO 2).

### PCR amplification of locus P-AC

The PCR reaction was prepared using the following reagents:

| *Reagent* | *Final concentration* |
|---|---|
| DNA | 10 ng |
| *Forward* primer (SEQ ID NO 7) | 200 nM |
| *Reverse* primer (SEQ ID NO 8) | 200 nM |
| MgCl₂ | 2 mM |
| dNTPs | 200 mM |
| PCR buffer (10 mM Tris-HCl pH 9.0, 50 mM KCI, 0.1 % Triton X-100) | 1x |
| Taq DNA polymerase | 1 unit |
| H₂O | qb to25 µl |

The amplification conditions include an initial denaturation at 94°C (4 min), followed by 10 cycles which comprise a denaturation at 94°C (40 sec), an annealing at a temperature decreasing by half a degree in each cycle from 55 °C to 50°C (40 sec) and an extension at 72°C (40 sec). The amplification proceeds with 25 denaturation cycles at 94°C (40 sec), annealing at 50 °C (40 sec) and extension at 72 °C (40 sec), ending with a final extension at 72 °C for 7 minutes.

The amplification products were separated by electrophoresis on 2% agarose gel. In the polynucleotide of SEQ ID NO 5 the VNTR is repeated 3 times, but in the different varieties of *Coffea arabica,* whose origin and genealogy is shown in table 3 below, said VNTR is repeated 2, 3 or 4 times, whereas it is always present as a single copy in *Coffea canephora* (figure 3).

**Table 3: Varieties of Coffea arabica, interspecies hybrids and other species**

| **Plant** | **Genealogy** | **Origin** |
|---|---|---|
| Typica | Variety of *C. arabica* | Costa Rica, CATIE |
| Bourbon VD | Mutant of Bourbon | Brazil, Patrocinio |
| Caturra | Mutant of Bourbon | Costa Rica, CATIE |
| Laurina | Mutant of Bourbon | Brazil, Patrocinio |
| Etypica | Ethiopian selection of *C*. *arabica* | Brazil, Patrocinio |
| Tafarikela Sln 4 | Wild selection of *C. arabica* | India, Karnataka Chikmagalur |
| Agaro-1 (1-24) | *C. arabica* from Ethiopia | Agaro, Ethiopia |
| Agaro-2 (1-24) | *C. arabica* from Ethiopia | Agaro, Ethiopia |
| Agaro-3 (1-24) | *C. arabica* from Ethiopia | Agaro, Ethiopia |
| Yirga (4, 8) | *C. arabica* from Ethiopia | Yirgacheffè, Ethiopia |
| Catuai 144 | Mundo Novo x Caturra | Brazil, Patrocinio |
| Catuai | Mundo Novo x Caturra | Brazil, Patrocinio |
| Aramosa | *C. arabica x C. racemosa* | Brazil, Patrocinio |
| Hibrido de Timor (HDT) | *C. arabica x C. canephora* | Costa Rica, CATIE |
| Sln.5B | S333 x Devamachy | India, Karnataka Chikmagalur |
| *C. canephora* T3481 t5 | Species | Costa Rica, CATIE |
| *C. canephora* T3483t1 | Species | Costa Rica, CATIE |
| *C. canephora* T3518t2 | Species | Costa Rica, CATIE |
| *C. canephora* T3563t2 | Species | Costa Rica, CATIE |
| *C. canephora* T3564t1 | Species | Costa Rica, CATI E |
| *C. eugenioides* | Species | Brazil, Campinas |

Figure 4 shows the amplification products in different roasted and ground commercial samples of which, in many cases, the composition is unknown. It can be seen that, in the case of products sold as Arabica coffee, the analysis confirms the seller's claims. In the other samples examined, where the composition is not declared on the package, it is seen to vary from pure Arabica to pure Robusta and blends thereof.

## Claims

1. A molecular method for distinguishing between different species of coffee (*Coffea spp.)* and hybrids thereof comprising the amplification of polymorphic sites defined by an insertion of sequence SEQ ID NO:2 and by a repetition of sequence SEQ ID NO:6 from 1 to n times, where n is up to 4, wherein the SEQ ID NO:2 is an insertion sequence comprised or not comprised within an isolated sequence of the nuclear chromosomal *locus* RM-00-O18 identified by SEQ ID NO:1 and the sequence SEQ ID NO:6 is a tandem repeat of a microsatellite within an isolated sequence of the plastidic *locus* P-AC identified by SEQ ID NO:5.

2. The method according to claim 1, wherein n is 1, 2, 3 and 4.

3. The method according to claims 1 and 2 comprising:
a) amplification by means of a polymerase chain reaction of a DNA fragment from a coffee DNA sample by means of oligonucleotide primers comprised between nucleotides 1-87 and 88-261 of the nuclear chromosome *locus* RM-00-O18 (SEQ ID NO:1) and by means of oligonucleotide primers comprised between nucleotides 1-392 and 393-864 of the plastidic chromosome *locus* P-AC (SEQ ID NO:5);
b) separation of the amplified fragment according to the amplification product molecular weight;
c) comparison of the previously identified molecular weight with that of the fragments generated from the same amplification type in the reference species/cultivar.

4. The method according to claim 3, wherein for *locus* RM-00-O18 the oligonucleotide primers are selected from nucleotides 27-47 and 235-259 of the sequence SEQ ID NO:1.

5. The method according to claim 3, wherein for *locus* P-AC the oligonucleotide primers are selected from nucleotides 355-375 and 408-427 of the sequence SEQ ID NO:5.

6. The method according to claim 3, wherein said comparison identifies in the amplified products obtained in a) the presence/absence of at least one characteristic fragment of a determined species or *cultivar of* the RM-00-O18 *locus* and a difference of size thereof of the P-AC *locus.*

7. The method according to claims 3-6, wherein the following primer pairs are used for the polymerase chain reaction: SEQ ID NO:3 and SEQ ID NO:4, and SEQ ID NO:7 and SEQ ID NO:8.

8. An isolated polynucleotide of coffee consisting of the sequence SEQ ID NO:1 or a sequence complementary thereof.

9. An isolated oligonucleotide of coffee consisting of the sequence SEQ ID NO:2.

10. Poly- and oligo-nucleotides derived from the sequences comprised between nucleotides 1 and 87 and nucleotides 88 and 261 of the sequence SEQ ID NO:1.

11. Poly- and oligo-nucleotides according to claim 10, wherein the sequences are comprised between nucleotides 27 and 47 and nucleotides 235 and 259 of the sequence SEQ ID NO:1.

12. Oligonucleotides according to claim 11 having SEQ ID NO:3 and SEQ ID NO:4.

13. A kit for amplification of chromosomal DNA fragments of coffee species (*Coffea spp.)* and hybrids thereof comprising an oligonucleotide pair consisting of a *forward* oligo- and a *reverse* oligo- primer for the PCR derived from the sequence SEQ ID NO:1 and an oligo-nucleotide pair consisting of a *forward* oligo-and a *reverse* oligo- primer for the PCR derived from the sequence SEQ ID NO:5 and optionally, as amplification control sequences, a polynucleotide of the *locus* RM-00-O18 and a polynucleotide of the *locus* P-AC.

14. The kit according to preceding claim comprising pairs of oligonucleotides consisting of a *forward* oligo- and a *reverse* oligo- primer for the PCR of sequence SEQ ID NO:3 and SEQ ID NO:4 and sequence SEQ ID NO:7 and SEQ ID NO:8.

## Patentansprüche

1. Molekulares Verfahren zum Unterscheiden zwischen verschiedenen Spezies von Kaffee (*Coffea spp.)* und Hybriden davon, umfassend die Amplifikation von polymorphen Sites, definiert durch eine Insertion einer Sequenz mit der Seq.-ID Nr. 2 und durch eine 1- bis n-malige Repetition einer Sequenz mit einer Seq.-ID Nr. 6, worin n bis zu 4 ist, worin die Seq.-ID Nr. 2 eine Insertionssequenz ist, die besteht aus, oder - innerhalb einer isolierten Sequenz - nicht besteht aus, dem nuklearen chromosomalen *locus* RM-00-O18, identifiziert durch die Seq.-ID Nr. 1, und die Sequenz mit der Seq.-ID Nr. 6 ein Tandem-Repeat eines Mikrosatelliten innerhalb einer isolierten Sequenz des plastidischen *locus* P-AC, identifiziert durch Seq.-ID Nr. 5 ist.

2. Verfahren nach Anspruch 1, worin n 1, 2, 3 und 4 ist.

3. Verfahren nach den Ansprüchen 1 und 2, umfassend
(a) ein Amplifizieren eines DNS-Fragments von einer Kaffee-DNS-Probe mittels einer Polymerase-Ketten-Reaktion mittels Oligonukleotid-Primern, die bestehen aus Oligonukleotiden zwischen den Nukleotiden 1 bis 87 und 88 bis 261 des nuklearen Chromosomen-*locus* RM-00-O18 (Seq.-ID Nr. 1), und mittels Oligonukleotid-Primern, die bestehen aus Oligonukleotiden zwischen den Nukleotiden 1 bis 392 und 393 bis 864 des plastidischen Chromosomen-*locus* P-AC (Seq.-ID Nr. 5);
(b) ein Auftrennen des amplifizierten Fragments nach dem Molekulargewicht des Amplifizierungs-Produkts;
(c) ein Vergleichen des vorher identifizierten Molekulargewichts mit dem der aus demselben Amplifizierungs-Typ erzeugten Fragmente in der Referenz-Spezies/in dem Referenz-Kultivar (der Referenz-Sorte).

4. Verfahren nach Anspruch 3, worin für den *locus* RM-00-O18 die Oligonukleotid-Primer gewählt sind aus Nukleotiden 27 bis 47 und 235 bis 259 der Sequenz Seq.-ID Nr. 1.

5. Verfahren nach Anspruch 3, worin für den *locus* P-AC die Oligonukleotid-Primer gewählt sind aus Nukleotiden 355 bis 375 und 408 bis 427 der Sequenz Seq.-ID Nr. 5.

6. Verfahren nach Anspruch 3, worin der Vergleich in den amplifizierten Produkten, die in Schritt (a) erhalten werden, das Vorhandensein/die Abwesenheit von wenigstens einem charakteristischen Fragment des RM-00-O18*-locus* oder einen Unterschied der Größe davon des P-AC*-locus* einer vorbestimmten Spezies oder eines Kultivars/einer Sorte identifiziert.

7. Verfahren nach den Ansprüchen 3 bis 6, worin die folgenden Primer-Paare für die Polymerase-Ketten-Reaktion verwendet werden: Seq.-ID Nr. 3 und Seq.-ID Nr. 4, und Seq.-ID Nr. 7 und Seq.-ID Nr. 8.

8. Isoliertes Polynukleotid von Kaffee, bestehend aus der Sequenz Seq.-ID Nr. 1 oder einer dazu komplementären Sequenz.

9. Isoliertes Oligonukleotid von Kaffee, bestehend aus der Sequenz Seq.-ID Nr. 2.

10. Polynukleotide und Oligonukleotide, abgeleitet aus den Sequenzen bestehend aus Nukleotiden zwischen den Nukleotiden 1 und 87 und den Nukleotiden 88 bis 261 der Sequenz Seq.-ID Nr. 1.

11. Polynukleotide und Oligonukleotide nach Anspruch 10, worin die Sequenzen bestehen aus Nukleotiden zwischen den Nukleotiden 27 und 47 und den Nukleotiden 235 und 259 der Sequenz Seq.-ID Nr. 1.

12. Oligonukleotide nach Anspruch 11, aufweisend die Seq.-ID Nr. 3 und Seq.-ID Nr. 4.

13. Kit für ein Amplifizieren von chromosomalen DNS-Fragmenten von Kaffee-Spezies (*Coffea spp.)* und Hybriden davon, umfassend ein Oligonukleotid-Paar bestehend aus einem *forward*-Oligo-Primer und einem *reverse*-Oligo-Primer für die PCR, abgeleitet von der Sequenz Seq.-ID Nr. 1, und ein Oligonukleotid-Paar bestehend aus einem *forward*-Oligo-Primer und einem *reverse*-Oligo-Primer für die PCR, abgeleitet von der Sequenz Seq.-ID Nr. 5, und gegebenenfalls einem Polynukleotid des *locus* RM-00-O18 und einem Polynukleotid des *locus* P-AC als Amplifizierungs-Steuer-Sequenzen.

14. Kit nach dem vorangehenden Anspruch, umfassend Paare von Oligonukleotiden, die bestehen aus einem *forward*-Oligo-Primer und einem *reverse*-Oligo-Primer für die PCR mit der Sequenz der Seq.-ID Nr. 3 und der Seq.-ID Nr. 4, und der Sequenz der Seq.-ID Nr. 7 und der Seq.-ID Nr. 8.

## Revendications

1. Procédé moléculaire pour faire la distinction entre différentes espèces de café *(Coffea spp.)* et leurs hybrides, comprenant l'amplification de sites polymorphes définis par une insertion de séquence SEQ ID NO : 2 et par une répétition de séquence SEQ ID NO : 6 de 1 à n fois, où n va jusqu'à 4, dans lequel SEQ ID NO : 2 est une séquence d'insertion comprise ou non comprise au sein d'une séquence isolée du locus chromosomique nucléaire RM-00-O18 défini par SEQ ID NO : 1 et la séquence SEQ ID NO : 6 est une répétition en tandem d'un microsatellite au sein d'une séquence isolée du locus plastidique P-AC identifié par SEQ ID NO : 5.

2. Procédé selon la revendication 1, dans lequel n est 1, 2, 3 et 4.

3. Procédé selon les revendications 1 et 2, comprenant :
a) une amplification, par amplification en chaîne par polymérase, d'un fragment d'ADN provenant d'un échantillon d'ADN de café au moyen d'amorces oligonucléotidiques comprises entre les nucléotides 1 à 87 et 88 à 261 du locus chromosomique nucléaire RM-00-O18 (SEQ ID NO : 1) et au moyen d'amorces oligonucléotidiques comprises entre les nucléotides 1 à 392 et 393 à 864 du locus chromosomique plastidique P-AC (SEQ ID NO : 5) ;
b) la séparation du fragment amplifié en fonction du poids moléculaire du produit d'amplification ;
c) la comparaison du poids moléculaire précédemment identifié avec celui des fragments générés à partir du même type d'amplification dans l'espèce/cultivar de référence.

4. Procédé selon la revendication 3, dans lequel, pour le locus RM-00-O18, les amorces oligonucléotidiques sont choisies parmi les nucléotides 27 à 47 et 235 à 259 de la séquence SEQ ID NO : 1.

5. Procédé selon la revendication 3, dans lequel, pour le locus P-AC, les amorces oligonucléotidiques sont choisies parmi les nucléotides 355 à 375 et 408 à 427 de la séquence SEQ ID NO : 5.

6. Procédé selon la revendication 3, dans lequel ladite comparaison identifie dans les produits amplifiés obtenus dans l'étape a) la présence/absence d'au moins un fragment caractéristique d'une espèce ou d'un cultivar déterminé du locus RM-00-O18 et une différence de taille de celui-ci du locus P-AC.

7. Procédé selon les revendications 3 à 6, dans lequel les paires d'amorces suivantes sont utilisées pour l'amplification en chaîne par polymérise : SEQ ID NO : 3 et SEQ ID NO : 4, et SEQ ID NO : 7 et SEQ ID NO : 8.

8. Polynucléotide isolé de café constitué de la séquence SEQ ID NO : 1 ou d'une séquence complémentaire de celle-ci.

9. Oligonucléotidique isolée de café constitué de la séquence SEQ ID NO : 2.

10. Poly- et oligo-nucléotides dérivés des séquences comprises entre les nucléotides 1 et 87 et les nucléotides 88 et 261 de la séquence SEQ ID NO : 1.

11. Poly- et oligo-nucléotides selon la revendication 10, dans lesquels les séquences sont comprises entre les nucléotides 27 et 47 et les nucléotides 235 et 259 de la séquence SEQ ID NO : 1.

12. Oligonucléotides selon la revendication 11, ayant SEQ ID NO : 3 et SEQ ID NO : 4.

13. Kit pour l'amplification de fragments d'ADN chromosomique d'espèces de café *(Coffea spp.)* et de leurs hybrides, comprenant une paire d'oligonucléotides constituée d'une amorce oligonucléotidique sens et d'une amorce oligonucléotidique antisens pour la PCR, dérivées de la séquence SEQ ID NO : 1, et une paire d'oligonucléotides constituée d'une amorce oligonucléotidique sens et d'une amorce oligonucléotidique antisens pour la PCR, dérivées de la séquence SEQ ID NO : 5, et éventuellement, en tant que séquence de régulation de l'amplification, un polynucléotide du locus RM-00-O18 et un polynucléotide du locus P-AC.

14. Kit selon la revendication précédente, comprenant des paires d'oligonucléotides constituées d'une amorce oligonucléotidique sens et d'une amorce oligonucléotidique antisens pour la PCR de séquences SEQ ID NO : 3 et SEQ ID NO : 4 et de séquences SEQ ID NO : 7 et SEQ ID NO : 8.


### SEQUENCE LISTING

<110> UNIVERSITA' DEGLI STUDI DI TRIESTE
<120> Method for distinguishing between the species Coffea arabica and Coffea canephora and hybrids thereof based on the concomitant analysis of nuclear and chloroplastic DNA polymorphisms
<130> 8840PTWO
<150> PD2008A000307
   <151> 2008-10-27
<160> 8
<170> PatentIn version 3.5
<210> 1
   <211> 261
   <212> DNA
   <213> Coffea arabica
<220>
   <221> misc_feature
   <222> (1)..(261)
   <223> polymorphic insertion after nucletide 87 (starting at position 88)
<400> 1
   cgtcaaggga ggaaaagaga agggtttgga aatggttaag gctgttgagg cttacctcca      60
   cgcaaatcct actgcctaca actaattata ttttcttcaa gattgaagta taaactgagg     120
   gtgtctgttt ccttttttgc ttggtttgct ttaagctgtt tggttgagtt atttgcagct     180
   atgatcataa tatttgatcg aagtgtgctt ttcttgcaac ctttgtatcg ttacaaggaa     240
   taaaatcttg tttggagctt t                                               261
<210> 2
   <211> 30
   <212> DNA
   <213> Coffea arabica
<220>
   <221> misc_feature
   <222> (1)..(30)
   <223> insertion, repetition or deletion in RM018
<400> 2
   ataatgaatg ctactgaagg actcttcgac 30
<210> 3
   <211> 21
   <212> DNA
   <213> Coffea arabica
<220>
   <221> misc_feature
   <222> (1)..(21)
   <223> PCR primer forward
<400> 3
   tggaaatggt taaggctgtt g 21
<210> 4
   <211> 25
   <212> DNA
   <213> Coffea arabica
<220>
   <221> misc_feature
   <222> (1)..(25)
   <223> PCR primer reverse
<400> 4
   agctccaaac aagattttat tcctt 25
<210> 5
   <211> 864
   <212> DNA
   <213> Coffea arabica
<220>
   <221> misc_feature
   <222> (1)..(864)
   <223> Chloroplast DNA: polymorphic insertion /repetition at position 393 (after nucleotide 392)
<400> 5
   tcacaaaata aaaagaaaag ccattccatt tcgacaaaag accgacaccc aagttccata     60
   gcttttggtc cgctatcccc accattctac ccccagaggg aagggtactt cccccttggg   120
   ccggttgtgg gcgaggaggg attcgaaccc ccgacaccgt ggttcgtagc cacgtgctct    180
   aatcctctga gctacaggcc ccacctcgtc tccactggat atgttaccgg gagtaccctc    240
   aaaaaaggga ctcctctccc cagccatttc gggttaagaa gatgtgaaag cgcgtttatc    300
   tctataagaa cggtgcgttc cgaggtgtga agtgggagag aagggatgtc ataattgggg    360
   ttttgaataa gacgaccttt ttctttttca ttaatgagag gtcttaagct ttttatcatc    420
   ctggcgtcga gctatttttc cgcaggacct cccctacagt atcgtcaccg cagtagagtt    480
   taaccaccaa gttcgggatg gattggtgtg gttcctctac gcctaggaca ccagaatatc    540
   gaatcctgaa cgaagaaagg catgagagaa aagtatattg gctagtgatt atgaggtccc    600
   aattcttgac tgggggggac accaaaggcc tctgcccttc catcccttag atagagaggg    660
   agggcggagc ttttggtttt ttcatgttgt caaagagttg aacaatgttt tttcatgttg    720
   tcaaagattt gaacaatgaa aatagatggc gagtgcctga tcgaattgat caggtcatgt    780
   aggaacaagg ttcaagtcta ccggtctgtt aggatgcctc agctgcatac atcactgcac    840
   ttccacttga cacctatcgt aatg                                           864
<210> 6
   <211> 32
   <212> DNA
   <213> Coffea arabica
<220>
   <221> satellite
   <222> (1)..(32)
   <223> insertion repetition or deletion
<400> 6
   ttttcgtttt catatattga aaaagtaata ag 32
<210> 7
   <211> 21
   <212> DNA
   <213> Coffea arabica
<220>
   <221> misc_feature
   <222> (1)..(21)
   <223> forward primer P-AC
<400> 7
   ttggggtttt gaataagacg a 21
<210> 8
   <211> 20
   <212> DNA
   <213> Coffea arabica
<220>
   <221> misc_feature
   <222> (1)..(20)
   <223> PCR primer reverse
<400> 8
   acgccaggat gataaaaagc 20